**Europäisches Patentamt**

**European Patent Office**

**Office européen des brevets**

(19)

(11) Veröffentlichungsnummer : **0 113 896 B2**

(12) ## NEUE EUROPÄISCHE PATENTSCHRIFT

(45) Veröffentlichungstag der neuen Patentschrift :
**11.11.92 Patentblatt 92/46**

(51) Int. Cl.⁵ : **G01N 33/52, C12Q 1/00**

(21) Anmeldenummer : **83112680.0**

(22) Anmeldetag : **16.12.83**

(54) **Teststreifen.**

Die Akte enthält technische Angaben, die nach dem Eingang der Anmeldung eingereicht wurden und die nicht in dieser Patentschrift enthalten sind.

(30) Priorität : **23.12.82 DE 3247608**

(43) Veröffentlichungstag der Anmeldung :
**25.07.84 Patentblatt 84/30**

(45) Bekanntmachung des Hinweises auf die Patenterteilung :
**15.10.86 Patentblatt 86/42**

(45) Bekanntmachung des Hinweises auf die Entscheidung über den Einspruch :
**11.11.92 Patentblatt 92/46**

(84) Benannte Vertragsstaaten :
**AT BE CH DE FR GB IT LI LU NL SE**

(56) Entgegenhaltungen :
EP-A- 0 064 710
DE-A- 2 752 352
DE-A- 2 825 636
DE-A- 2 910 134
DE-A- 3 130 749
DE-B- 2 158 124
DE-C- 1 598 153

(56) Entgegenhaltungen :
US-A- 3 607 093
US-A- 3 802 842
US-A- 3 901 657
US-A- 4 015 462
US-A- 4 046 513
US-A- 4 160 008
US-A-40 614 68
US-A-42 159 95
Ullmanns Enzyklopädie der technischen Chemie, 4. Auflage, Band 2, Seite 195 und Band 16, Seiten 515 ff.
Meyers Lexikon der Technik und der exakten Naturwissenschaften, Bibliographisches Institut, Mannheim/Wien/Zürich. Seiten891,892, 1896 und 2671, zweiter Band.

(73) Patentinhaber : **BOEHRINGER MANNHEIM GMBH**
**Sandhofer Strasse 116**
**W-6800 Mannheim 31 (DE)**

(72) Erfinder : **Rothe, Anselm, Dr.rer.nat.**
**Tiefenklingerweg 21**
**W-6943 Birkenau (DE)**
Erfinder : **Knappe, Wolfgang-Reinhold, Dr.rer.nat.**
**Karlsbader Strasse 3**
**W-6842 Bürstadt 1 (DE)**
Erfinder : **Trasch, Heinz-Friedrich, Dr.rer.nat.**
**Wissmannstrasse 14**
**W-6700 Ludwigshafen (DE)**

EP 0 113 896 B2

**Beschreibung**

Die Erfindung betrifft neue Teststreifen aus Reagenzien enthaltenden Filmschichten auf einer multifilen Gewebe-Trägerschicht sowie Verfahren zur ihrer Herstellung und ihre Verwendung.

Testpapiere, das heißt mit Reagenzien imprägnierte Papierstreifen, werden in der analytischen Chemie seit langem zum Nachweis von anorganischen Ionen, organischen Substanzen und zur pH-Bestimmung in Flüssigkeiten und Gasen verwendet. Eine Weiterentwicklung sind die Teststreifen oder Teststäbchen, bei denen das imprägnierte Reagenzpapier auf einer als Griff dienenden Kunststoffolie befestigt ist. Diese gewinnen in der analytischen Chemie, wie der Produktkontrolle, der Wasser- und Abwasseruntersuchumg und insbesondere in der klinischen Chemie bei der Untersuchung von Körperflüssigkeiten steigende Bedeutung. Bisher waren dabei die Reagenzien üblicherweise auf Papier imprägniert. Solche Teststreifen erlaubten eine rasche, aber nur semiquantitative visuelle Bestimmung, mittels entsprechender Farbvergleichsfelder. Die natürlichen Ungleichmäßigkeiten der Papiere umd die dadurch bedingte ungleichmäßige Streuung des Lichts erschwerten eine quantitative, remissionsphotometrische Auswertung.

Mit dem Bedarf nach quantitativen Meßergebnissen unter Anwendung der Teststreifen auch auf die Blut- und Serenanalytik wurde zunehmend die Verwendung von Reagenzfilmen üblich, deren Hauptvorzug die hohe Gleichmäßigkeit der Reagenzienschicht ist. Durch die Verwendung von Reagenzienfilmen und remissionsphotometrische Auswertung der Farbreaktion ist es nunmehr möglich, mit Teststreifen Meßergebnisse in der Qualität der naßchemischen Methoden zu erzielen.

Beispiele für Teststreifen auf Filmbasis sind beschrieben in der DE-AS 15 98 153, zur Bestimmung von vorzugsweise Glucose im Blut, oder in der DE-OS 31 18 381, in der ein Teststreifenaufbau beschrieben ist, der zur Bestimmung von Glucose in Harn die Verwendung eines solchen Reagenzienfilmes auch bei undosiertem Eintauchen in die Probeflüssigkeit ermöglicht. In der DE-OS 31 30 749 schließlich werden Teststreifen zur Bestimmung von Blutparametern beschrieben, die zusätzlich zum Reagenzfeld - vorzugsweise auf Filmbasis - eine vorgeschaltete Erythrozytenabtrennung mittels eines Glasfaservlieses besitzen und eine Analyse direkt aus Vollblut erlauben.

Der Reagenzfilm ist bei diesen Teststreifen entweder direkt auf der als Griff dienenden Kunststoffolie angebracht oder befindet sich auf einer zusätzlichen Trägerfolie, die ihm die bei der Herstellung und Verarbeitung nötige Stabilität verleiht.

Bei Reagenzienfilmen, die im Vergleich zu den meist flauschigen und "offenen" Papieren eine glatte Oberfläche und einen nur geringen Anteil an Hohlräumen besitzen, finden sich jedoch aus eben diesen Gründen auch gewisse Nachteile.

So kann es bei Teststreifen mit relativ geschlossenem Aufbau, wie sie in DE-OS 31 30 749 beschrieben sind, Schwierigkeiten bereiten, einer sauerstoffverbrauchenden Reaktion den dafür nötigen Sauerstoff in der geforderten kurzen Reaktionszeit ausreichend rasch zuzuführen. Die dort beschriebenen Reaktionsschichten stehen nämlich nicht in direktem Kontakt mit der Luft, sondern werden auf ihrer Oberseite durch ihre sauerstoffundurchlässige, durchsichtige Trägerfolie von vornherein abgeschlossen und ebenfalls auf ihrer Unterseite mach Andruck der Reagenzeinschicht auf das Blutabtrennvlies.

Um eine ausreichende Sauerstoffversorgung zu gewährleisten, ist es deshalb nötig, durch aufwendige Maßnahmen im Meßgerät ein zeitweises Wiederabheben der Reagenzienschicht vom Trennvlies vorzusehen. Dies übt zudem einen umgünstigen Einfluß auf die Qualität der Meßwerte aus. Auch bei dem obenerwähnten Harnteststreifen gemäß DE-OS 31 18 381 ergaben sich zwei Phänomene, die auf die Eigenschaften von Reagenzienfilmen zurückzuführen sind. Bei diesen Harnteststreifen zur Bestimmung von Glucose wird der Reagenzienfilm auf seiner Trägerfolie über ein verzögert saugendes Papier mittels eines dünnen Abdecknetzes auf der Griffolie befestigt. Es zeigte sich, daß bei unsachgemäßer Handhabung bisweilen eine Luftblase zwischen Abdecknetz und Reagenzfilmoberfläche eingeschlossen blieb und eine Farbreaktion verhinderte. Andererseits konnte es vorkommen, daß beim Absaugen größerer Mengen überschüssigen Harns ins verzögernd saugende Papier an den Absaugkanten unschöne Farbränder entstanden, die zu Fehlinterpretationen führten.

Die Luftblasenbildung konnte nur durch eine kostspielige Netzmittelbehamdlung des Abdecknetzes verhindert werden, eine Lösung des Farbrandproblems war nur durch eine Beschränkung auf kleine Testfelformate möglich.

Es wurde nun überraschend gefunden, daß sich die eben genannten Probleme einfach und vollständig dadurch beheben lassen, daß man Teststreifen aus einer Reagenzien enthaltenden Filmschicht und einer als Griff dienenden Kunststoffolie verwendet, bei denen sich der Reagenzfilm überwiegend auf einer Seite einer als multifiles Gewebe oder Vlies ausgebildeten Trägerschicht befindet. Der Reagenzienfilm wird somit nicht auf eine feste Trägerfolie, sondern auf eine als multifiles Gewebe oder Vlies ausgebildete Trägerschicht, vorzugsweise aus Polyester oder Polyamid, beschichtet. Zu diesem Zweck wird eine möglichst konzentrierte Suspension beziehungsweise Dispersion der filmbildenden Kunststoffe, der notwendigen Reagenzien, Pigmente

2

sowie sonstiger Füll- und getrocknet. Es ergeben sich trockene Filme von 10 - 200 μm, vorzugsweise 15 - 100 μm. Es ist kennzeichnend für die Erfindung, daß die Beschichtungsmasse im wesentlichen auf der Oberseite des Gewebes verbleibt, während ins Innere des Gewebes und an seine Unterseite je nach Auftragsmenge und Massenbeschaffenheit wenig bis überhaupt keine Beschichtungsmasse gelangt. Die Verwendung der so hergestellten Reagenzfilme zur Herstellung der vorbezeichneten Teststreifen ist ebenfalls Gegenstand der Erfindung. Dazu wird der Reagenzfilm in an sich bekannter Weise mit einer als Griff dienenden Kunststoffolie verklebt oder mittels eines dünnen Netzes, welches den Reagenzfilm überdeckt und seitlich davon mit der Griffolie verbunden ist, befestigt. Zwischen Griffolie und Reagerzfilm kann zusätzlich noch eine saugfähige Schicht vorhanden sein. Die Filmschicht kann auch durch eine ggf. durchsichtige Abdeckschicht bedeckt sein. Selbstverständlich sind auch andere Vliese oder Gewebe aus anderen Rohstoffen, wie Bauwolltuch, geeignet, solange sie den Anforderungen an Gleichmäßigkeit, Saugvermögen und Durchlässigkeit genügen.

Monofile Gewebe, deren Einbettung in Beschichtungsmassen in der DE-OS 28 25 636 beschrieben wurde, sind für die erfindungsgemäße Anwendung nicht geeignet. Einerseits Schlägt die Beschichtungsmasse während der Beschichtung durchs Gewebe durch, so daß eine zusätzliche, später zu verwerfende Trägerfolie eingesetzt werden muß. Andererseits tritt bei monofilen Geweben der beschleunigende Effekt bei sauerstoffverbrauchenden Reaktionen nicht auf. Schließlich sind gleichmäßige Filme erst mit relativ hohen Schichtdicken möglich und besitzen eine untragbar lange Reaktionszeit und auch nicht den erfindungsgemäßen typischen Unterschied zwischen Gewebeseite und Schichtseite.

Die für den Aufbau des Reagenzienfilms geeigneten Reagenzien, Filmbildner und Hilfsstoffe sind die gleichen, für Reagenzfilme gebräuchlichen, wie sie z.B. in den DE-AS 15 98 153, DE-OS 29 10 134 oder DE-OS 31 18 381 beschrieben sind.

Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, z.B. von Butadien und Styrol oder von Maleinsäureester und Vinylacetat, Cellulose und Cellulosederivate oder Gelatine. Es können jedoch auch andere Hilfsstoffe in geeigneten Lösungsmitteln, vorzugsweise Wasser, mit einer Rakel oder Düse in dünner Schicht (20 - 500 μm, vorzugsweise 50 - 200 μm) auf das Gewebe aufgebracht und filmbildende, natürliche synthetische organische Polymere sowie Mischungen derselben bevorzugt in Form von wäßrigen Dispersionen, verwendet werden Die Filmbildner können auch in organischen Lösungsmitteln gelöst sein, z.B. ein Copolymerisat aus Vinylchlorid und Vinylpropionat in Essigester.

Die für die Nachweisreaktion erforderlichen Reagenzien, Pigmente und sonstigen Hilfsstoffe, werden normalerweise direkt in die Dispersion gegeben. Sofern vorteilhaft, kann der gebildete Film aber mit ihnen auch imprägniert werden. Auch eine Vorimprägnierung der zugesetzten Pigmente mit den Reagenzien ist möglich. Die Verfahren lassen sich auch derart kombinieren, daß z.B. bestimme Bestandteile in die Dispersion gegeben werden und andere auf den Film nachimprägniert werden. Dadurch läßt sich eine gewisse, räumliche Trennung der Bestandteile erreichen, was zu stabileren oder reaktiveren Testen führen kann Weiterhin läßt sich eine Trennung dadurch bewirken, daß auf eine erste Filmschicht eine zweite mit unterschiedlicher Zusammensetzung aufgebracht wird.

Sofern nötig, lassen sich Verdickungsmitte, Emulgatoren, Dispergiermittel, Pigmente, Weichnacher und Netzmittel zusetzen.

Dispergiermittel, Emulgatoren und Verdickungsmittel dienen zum Herstellen und Stabilisieren der Dispersionen. Pigmente, wie z.B. Titandioxid, Siliziumdioxid verbessern die Remissionseigenschaften von Filmen, indem sie für möglichst geringe Transparenz und erhöhte Remission der Filme sorgen. Dies ist insbesondere dann von Vorteil, wenn die so erhaltenen diagnostischen Prüfmittel remissionsphotometrisch ausgewertet werden sollen.

Mit Weichmachern lassen sich die Eigenschaften der Filmbeschichtungsmassen sowie der Filme optimieren. Z.B. werden ihre Standfestigkeit, ihre Viskosität, ihre Haftung auf der zu beschichtenden Unterlage unter anderem verbessert.

Netzmittel werden eingesetzt, um eine bessere Benetzung des Films durch das Probengut zu erreichen. Sie können gleichzeitig aüch Reaktionen katalysieren oder Rezepturen stabilisieren oder die Reaktionsfarbe brillanter und kontrastreicher machen.

Die hierin beschriebenen Teststreifen werden vorzugsweise beim Nachweis der Inhaltsstoffe von Körperflüssigkeiten, wie Harn, Blut, Serum, Stuhlsaft und Speichel eingesetzt, können jedoch in geeigneter Modifizierung auch in anderen wässrigen Flüssigkeiten wie Trinkwasser, Abwasser und gegebenenfalls auch in organischen Lösungsmitteln, in denen sie unlöslich sind, eingesetzt werden.

Die erfindungsgemäß beschichteten Gewebe lassen sich in allen Fällen vorteilhaft einsetzen, in denen bisher eine mit einem Reagenzfilm beschichtete Folie eingesetzt wurde. Bevorzugt werden sie anstelle der Filme in Vorrichtungen gemäß DE-OS 31 18 381 umd 31 30 749 verwendet.

Bei Vorrichtungen gemäß DE-OS 31 30 749 erhält man durch die Verwendung von Gewebe als Unterlage überraschend eine raschere Reaktion bei sauerstoffverbrauchenden Reaktionen, möglicherweise weil durch die Gewebestruktur zusätzlich Sauerstoff bereitgestellt wird. Bei Vorrichtungen gemäß DE-OS 31 18 381 fließt ein eventueller Flüssigkeitsüberstand rascher und ohne Bildung von Farbfänder ab. Auch beim raschen Eintauchen oder ungenügendem Abstreifen ist der Kontakt zwischen Reagenzfilm und Abdecknetz so eng, daß sie keine störenden Luftblasen mehr ausbilden können. Die erfindungsgemäß beschichteten Gewebe lassen sich natürlich auch direkt auf die als Griff dienende Kunststoffolie aufkleben oder aufsiegeln, erfordern dann jedoch wie Teststreifen mit übhchen Reagenzfilmen auf Trägerfolien ein sorgfältiges Abstreifen überschüssiger Testlösung, da diese sonst auf der glatten Oberfläche in Tröpfen zurückbleibt und zu fleckigen Reaktionen führt. Unterlegung mit einem venögert saugfähigem Material bietet insofern keinen Vorteil, da die Flüssiget nur sehr langsam durch die Kunststoffschicht durchdringt.

Im Vergleich zu den auch heute noch überwiegend verwendeten Teststreifen mit imprägniertem Papier, bieten die filmbeschichteten Gewebe folgenden Vorteile:

Durch die Bestandteile von Beschichtungen (kollodiale Verdickungsmittel, Dispersionen) und relative Lösungsmittelarmut im Vergleich zu Tränklösungen können in homogener Lösung miteinander unverträglicher Reagenzien über mehrere Stunden hinweg nebeneinander stabil bleiben.

Durch dies Verwendung von pigmentierten Beschichtungsmassen ist für die remissionsphotometrische Auswertung ein Hintergrund mit einem Weißgrad zu erzielen, der durch Tränkung eines Papiers nicht erreicht werden kann.

Im Gegensatz zu imprägnierten Papieren, bei denen die Inhomogenität des Papiers eine photometrische Auswertung stört, ist das beschichtete Gewebe homogen, da die Schicht im wesentlichen auf der Oberseite liegt und die gleiche Homogenität aufweist wie ein auf einer Folie aufgetragener Film. So sind kürzere Reaktionszeiten erzielbar, da ein dünner Film verglichen mit einem saugfähigen Träger (Papier) mit nur einem Bruchteil der Substratmenge beschickt wird, somit rasch reagiert und praktisch die ganze entstandene Reaktionsfarbe zum Meßergebnis beitragen kann. Andererseits besteht die Möglichkeit einer vorgeschalteten Reaktion, indem das Gewebe vor der Beschichtung mit einem zusätzlichen Reagenz getränkt wird oder die Testlösung über einen oder mehrere saugfähige Träger auf das Gewebe und den Reagenzfilm übertragen wird.

**Beispiel 1**

Herstellung eines Teststreifens zum Nachweis von Glucose im Blut

    35 KU Glucoseoxidase
    200 KU Peroxidase
    15 ml 0,5 m Phosphatpuffer pH 5
    0,3 g Na-Alginat
    25 g Polyvinylpropionat-Dispersion 50% in Wasser
    0,5 g Tetramethylbenzidin (3,3′,5,5,)
    0,2 g Phenylsemicarbazid
    1 g Dioctylnatriumsulfosuccinat
    6 ml Methoxyäthanol
    20 g Titandioxid
    35 ml Wasser

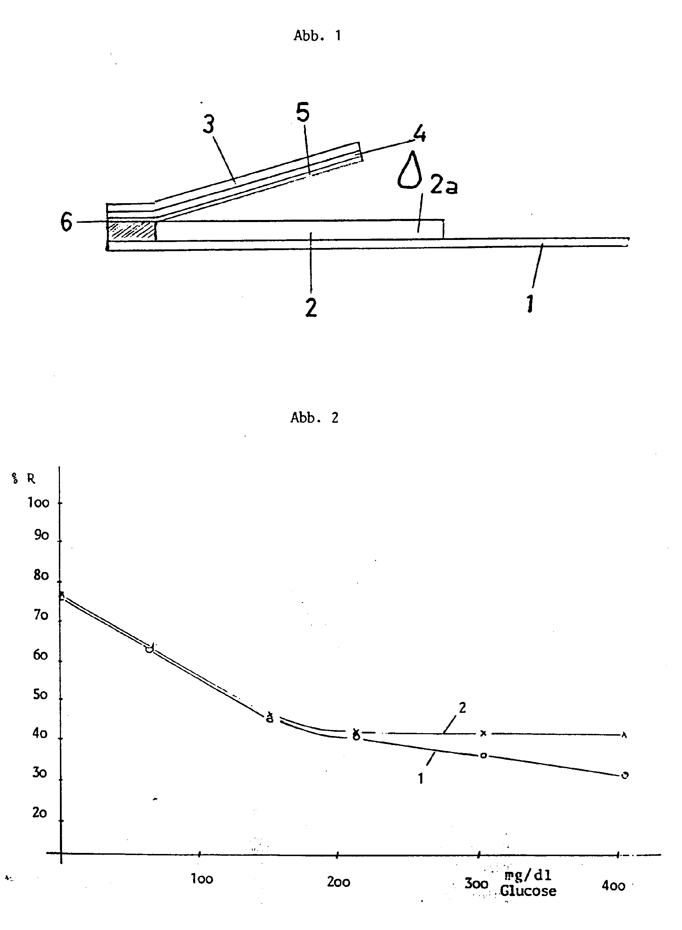

werden zu einer homogenen Masse verarbeitet und mit 0,1 mm Spaltbreite auf ein 150 μm dickes multifiles Polyamid-Gewebe (181 F 892 Schweizer Seidengaze-Fabrik) beschichtet und getrocknet. Das so erhaltene beschichtete Gewebe wird danach mit einer durchsichtigen Abdeckfolie verbunden, so daß die Abdeckfolie auf dem Reagenzfilm aufliegt. Anschließend wird ein 1 cm breiter Streifen dieses beschichteten Gewebes mit der Gewebeseite nach unten entsprechend Abb. 1 auf einem Plastikstreifen befestigt, auf dem bereits ein 15 mm breites Glasfaservlies, Dicke 1,5 mm, Faserdicke ca. 2 μm aufgebracht ist, so daß das freie Ende des beschichteten Gewebes noch 6 mm über das Vlies reicht. Dann wird in 6 mm breite Teststreifen geschnitten.

Bringt man nun 15 μl Vollblut auf den ProbenauftraBsbezirk 2a entsprechend Abb. 1, so durchdringt innerhalb 30 bis 60 Sekundem der Plasmaanteil das gesamte Glasfaservlies auch unterhalb der durchsichtigen Folie, während die Erythrozytem im Bereich 2a festgehalten werden. Durch Drückcn auf die Abdeckfolie kommt nun die Reagenzschicht über die Gewebeschicht mit dem abgetrennten Plasma in Berührung und wird gleichmäßig durchfeuchtet. Die im Plasma enthaltene Glucose reagiert innerhalb von 1 - 2 Min. in Abhängigkeit von ihrer Konzentration unter Entwicklung einer mehr oder weniger kräftigen Blaufärbung.

Die photometrische Messung geschieht vorzugsweise in einem Remissionsphotometer, dessen Meßkopf sich erst nach einer bestimmten Inkubationszeit (Plasmaabtrennung) auf das Testfeld senkt und mit dem damit erzeugten Druck den Kontakt zwischen Abtrennvlies und Reagenzgeweberückseite herstellt. Bei einer Reak-

tionszeit von 60 Sekunden und einer Meßwellenlänge von 630 nm erhält man die in Abb. 2, Kurve 1 dargestellte Meßkurve, Kurve 2 zeigt die Meßwerte, bei denen der Film direkt auf die Abdeckfolie als Trägerschicht aufgebracht ist und nicht auf ein Gewebe.

Der Unterschied von Kurve 1 und Kurve 2 zeigt deutlich, daß Abstufungen zu höchsten Glucosekonzentrationen erst durch den erfindungsgemäßen Aufbau ermöglicht werden. Die dabei erfolgende stärkere Reaktion, möglicherweise durch Sauerstoffzufuhr aus dem Inneren des Gewebes, war völlig unerwartet, da eine einfache Rechnung ergibt, daß der ursprünglich in den Gewebehohlräumen vorhandene Sauerstoff zur Deckung des Sauerstoffbedarfs der Reaktion bei weitem nicht ausreicht Außerdem ergab sich durch den erfindungsgemäßen Aufbau eine deutliche Verringerung der Reaktionszeit.

Die durchsichtige Abdeckfolie kann, im Gegensatz zu der in der DE-05 31 30 749 gezeigten Vorrichtung, entfallen, da die Reagenzschicht durch die darunterliegende Gewebeschicht ausreichend stabilisiert ist, kann jedoch aus Sicherheitsgründen zusätzlich vorhanden sein, um eine Berührung oder Beschädigung der Reagenzschicht zu verhindern.

**Legende zu Abb. 1**

1 Trägerfolie
2 Glasfaservlies
3 Abdeckfolie
4 Reagenzfilm
5 Gewebeschicht
6 Befestigung

**Beispiel 2**

Herstellung eines Teststreifens zum Nachweis von Glucose im Harn

20 KU Glucoseoxidase
80 KU Peroxidase
5 ml 1 M Citratpuffer pH 5
0,13 g Na-Alginat
13 g Poly inylpropionat-Dispersion 50%ig in
Wasser 0,375 g Tetramethylbenzidin (3,3′,5,5,)
0,1 g 1 -Phenylsemicarbazid
1 g Dioctylnatriumsulfosuccinat
5 ml Methoxyäthanol
10 g Kieselgel
12 ml Wasser

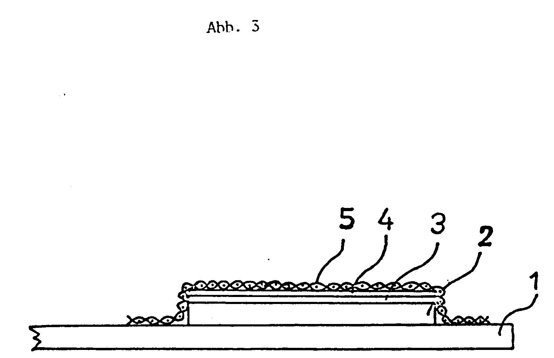

werden zu einer homogenen Masse verarbeitet und mit 0,1 mm Spaltbreite auf ein 350 μm dickes Polyester-Vlies (Dupont/Remey 2033) beschichtet und getrocknet Der so erhaltene beschichtete Träger wird wie in der DE-OS 31 18 381 beschrieben, über einem verzögert saugenden Papier auf einer als Griff dienenden Kunststoffolie mit einem netzmittelumbebandelten, dünnen Gewebe befestigt und zu Teststreifen verarbeitet (Abb. 3).

Taucht man die Streifen in glucosehaltige Harne, so ergeben sich auch bei nachlässiger Handhabung fleckenlose und randfreie Reaktionsfarben.

**Legende zu Abb. 3.**

1 Trägerfolie
2 saugfähiges Papier
3 Gewebeschicht
4 Reagenzfilm
5 Abdecknetz

**Beispiel 3**

Herstellung eines Teststreifens zum Nachweis von Cholesterin im Blut

2,5 KU Cholesterinoxidase
1,5 KU Cholesterinesterase
50 KU Peroxidase
10 mg Gallussäure
0,5 g Tetramethylbenzidin (3,3',5,5')
0,3 g Dioctylnatriumsulfosuccinat
1,5 ml Aceton
6,5 g Polyvinylpropionat-Dispersion 50%ig in Wasser
5 g Titandioxid
10 g Cellulose
15 ml Phosphatpuffer 0,5M, pH 7
20 ml Wasser

werden zu einer homogenen Masse verarbeitet und mit 0,15 mm Spaltbreite auf ein 200 µm dickes multifiles Polyester-Gewebe (2 F 777 Schweizer Seidengaze-Fabrik) beschichtet und getrocknet.

Der so erhaltene beschichtete Träger wird, wie im Beispiel 1 beschrieben, zu einem Teststreifem verarbeitet. Die Reaktion mit cholesterinhaltigem Blut geschah wie in Beispiel 1 und ergab nach 100 Sekunden Reaktionszeit eine ausgezeichnete Abstufung über den gesamten relevanten Konzentrationsbereich.

**Beispiel 4**

Herstellung eines Teststreifens zum Nachweis von Triglyceriden im Blut

50 KU Peroxidase
20 KU Cholesterinesterase
50 KU Glycerinkinase
10 KU Glycerophosphat-Oxidase
20 g Polyvinylpropionat-Dispersion 50%ig in Wasser
20 g Cellulose
0,2 g Na-Alginat
10 g Titandioxid
0,68 g Tetramethylbenzidin (3,3',5,5')
0,30 g Dioctylnatriumsulfosuccinat
1,5 ml Aceton
25 ml Phosphatpuffer 0,2M, pH 7,8
10 ml Wasser
0,2 g Adenosintriphosphat

werden zu einer homogenen Masse verarbeitet und mit 0,2 mm Spaltbreite auf ein 210 µm dickes Baumwollgewebe beschichtet und getrocknet. Der so erhaltene beschichtete Träger wird, wie in Beispiel 1 beschrieben, zu einem Teststreifen verarbeitet. Die Reaktion mit triglyceridhaltigem Blut geschah wie in Beispiel 1 und ergab nach 120 Sekunden Reaktionszeit eine ausgezeichnete Abstufung über den gesamten relevanten Konzentrationsbereich.

**Beispiel 5**

Herstellung eines Teststreifens zum Nachweis von Harnsäure im Blut

40 KU Peroxidase
1 KU Uricase
18 g Polyvinylpropionat-Dispersion 5o% in Wasser
0,25 g Na-Alginat
0,5 g nichtionogenes Netzmittel
0,05 g EDTA-Na$_2$
20 g Kieselgur
20 ml Phosphatpuffer 0,2m, pH 7

6

0,4 g Primaquin-diphosphat

18 ml Wasser

werden zu einer homogenen Masse verarbeitet und mit 0,2 mm Spaltbreite auf ein 200 µm dickes multifiles Polyester-Gewebe (2 F 777 Schweizer Seidengaze-Fabrik) beschichtet und getrocknet.

Mit

0,2 g 4-Aminoantipyrin

0,2 g nichtionogenes Netzmittel

in 50 ml Wasser

wird ein dünnes Filterpapier (597 NF-Ind., Schleicher ε Schüll) getränkt und getrocknet.

Es werden Teststreifen, wie in Beispiel 1 beschrieben, hergestellt, die zusätzlich zwischen Abtrennvlies und Reagenzgewebeunterseite eine Lage Aminoantipyrinpapier erhalten.

Die Reaktion mit harnsäurehaltigem Blut geschah wie in Beispiel 1 und ergab nach 120 Sekunden Reaktionszeit eine ausgezeichnete Abstufung über den gesamten relevanten Konzentrationsbereich.

**Beispiel 6**

Herstellung eines Teststreifens zum Nachweis von $\partial$-Glutamyltransferase im Blut

1,0 g N-Methylanthranilsäure

2,5 g Glycylglycin

0,85 g EDTA-$Na_2$

0,2 g Glutamyl-p-phenylendiamin-3-carbonsäure

20 g Polyvinylpropionat-Dispersion 50%ig in $H_2O$

0,2 g Na-Alginat

0,35 g Dioctylnatriumsulfosuccinat

1,0 ml Methanol

5 g Titandioxid

8 g Cellulose

15 ml Tris-Puffer, pH 7,6

15 ml Wasser

werden zu einer homogenen Masse verarbeitet und mit 0,15 mm Spaltbreite auf ein 250 µm dickes multifiles Polyamid-Gewebe (1093 Verseidag-Industrie-Textilien GmbH) beschichtet und getrocknet.

Mit 250 mmol/l $K_3[Fe(CN)_6]$ wird ein Teebeutelpapier der Fa. Schöller & Hösch, 12 g/ $m^2$ Flächengewicht inprägniert und 5 Minuten bei 30° C getrocknet. Es werden Teststreifen wie in Abb. 1 beschrieben hergestellt, die zusätzlich zwischen Abtrennvlies und Reagenzgewebeunterseite eine Lage des Oxidationspapiers erhalten.

Die Reaktion mit Gamma-GT-haltigen Blut geschah wie in Beispiel 1 und ergab nach 120 Sekunden Reaktionszeit eine ausgezeichnete Abstufung über den gesamten relevanten Konzentrationsbereich.

**Beispiel 7**

Herstellung eines Teststreifens zum Nachweis von Bilirubin im Blut

0,2 g 2-Methoxy-4-nitrobenzoldiazoniumtetrafluoroborat

1,5 g Metaphosphorsäure

1,5 g Diphenylphosporsäure

0,2 g Dioctylnatriumsulfosuccinat

5 g Kieselgel

1 g Cellulose

7,5 g Polyvinylidenchlorid-Dispersion (Diofan 217 D, BASF) 40%ig in Wasser

15 g Quellmittel (Bentone EW, National Lead) 2,5%ig in Wasser

werden zu einer homogenen Masse verarbeitet und mit 0,2 mm Spaltbreite auf ein 200 µm dickes multifiles Polyester-Gewebe (2 F 777 Schweizer Seidengaze-Fabrik) beschichtet und getrocknet.

Der so erhaltene beschichtete Träger wird wie in Beispiel 1 beschrieben zu einem Teststreifen verarbeitet. Die Reaktion mit bilirubinhaltigem Blut geschah wie in Beispiel 1 und ergab nach 60 Sekunden Reaktionszeit eine ausgezeichnete Abstufung über den gesamten relevanten Konzentrationsbereich.

**Beispiel 8**

Herstellung eines Teststreifens zum Nachweis von Harnsäure im Blut

8,4 g Gelatine
40 ml Phosphatpuffer (0,5 M; pH 7,0)
0,275 g Polyoxyethylensorbitanoleat (Tween 20)
5 ml Enzymsuspension (0,5 KU Uricase, 50 KU Peroxidase)
0,15 g 2(4-Hydroxy-3,5-dimethoxy-phenyl)-4,5-bis(p-dimethylaminophenyl)-imidazol-hydrochlorid)
0,25 ml Isopropanol

Die Reagenzien werden bei 37°C zu einer homogenen Masse verarbeitet und im zur Verarbeitung von Gelantinefilmen üblichen Vorhangverfahren über eine Schlitzdüse 300 µm auf ein multifiles Polyamidgewebe (Firma Schweizer Seidengazefabrik 2 F/131) aufgetragen und getrocknet.

Der so erhaltene Reagenzfilm wird wie im Beispiel 1 beschrieben zu Teststreifen gemäß Abb. 1 weiterverarbeitet.

Beim Aufbringen von ca. 300 µm Blut bei 37° C auf den Bezirk 2a und Aufpressen der Gewebeschicht (5) auf das Transportvlies (2) nach 1 - 2 Minuten, kann eine der Harnsäurekonzentration proportinale Blaufärbung nach weiteren 1 - 2 Minuten abgelesen werden. Mit einem Remissionsphotometer wird bei 680 nm die in Tab. 1 aufgeführte Remission gemessen.

**Tab. 1**

| Harnsäure mg/100 ml | % Remission |
|---|---|
| 0 | 66,4 |
| 5 | 46,7 |
| 15 | 33,4 |

**Patentansprüche**

1. Teststreifen mit einer Reagenzien enthaltenden Filmschicht und einer als Griff dienenden Kunststoffolie, **dadurch gekennzeichnet**, daß die Filmschicht zur Bildung eines filmbeschichteten Gewebes oder Vlieses durch Aufbringen und anschließendes Trocknen einer Reagenzien und einen Filmbildner enthaltenden Dispersion oder Lösung derartig auf einen Träger aus einem multifilen Gewebe oder Vlies beschichtet ist, daß sie sich überwiegend auf einer Seite desselben befindet und das filmbeschichtete Gewebe oder Vlies an der Griffolie befestigt ist.

2. Teststreifen nach Anspruch 1, dadurch gekennzeichnet, daß das Gewebe oder Vlies aus Polyester, Polyamid, Baumwolle oder Cellulose besteht.

3. Teststreifen nach einem der Ansprüche 1 - 2, dadurch gekennzeichnet, daß die trockene Filmschicht eine Dicke von 10 - 200 µm hat.

4. Teststreifen nach Anspruch 3, dadurch gekennzeichnet, daß die Filmschicht eine Dicke von 15 - 100 µm hat.

5. Teststreifen nach einem der Ansprüche 1 - 4, dadurch gekennzeichnet, daß sich zwischen der Griffolie und dem filmbeschichteten Gewebe oder Vlies eine zusätzliche saugfähige Schicht befindet.

6. Teststreifen nach Anspruch 5, dadurch gekennzeichnet, daß die saugfähige Schicht und das filmbeschichtete Gewebe oder Vlies mittels eines dünnen Netzes, welches neben dem Schichtpaket auf der Trägerfolie befestigt ist, verbunden sind.

7. Teststreifen nach Anspruch 5, dadurch gekennzeichnet, daß die Griffolie und die saugfähige Schicht miteinander verbunden sind

8. Teststreifen nach Anspruch 7, dadurch gekennzeichnet, daß sich oberhalb des filmbeschichteten Gewebes oder Vlieses eine Abdeckschicht befindet.

9. Teststreifen nach Anspruch 8, dadurch gekennzeichnet, daß die Abdeckschicht durchsichtig und mit dem filmbeschichteten Gewebe oder Vlies verbunden ist.

10. Verfahren zum Herstellen von Teststreifen gemäß einem der Ansprüche 1-9, **dadurch gekennzeichnet,** daß eine Dispersion bzw. Lösung der die Filmschicht bildenden Bestandteile in einem geeigneten Lösungsmittel auf eine Seite des Gewebes oder Vlieses aufgebracht und getrocknet wird, worauf diese in an sich bekannter Weise mit den übrigen Komponenten des Teststreifens verbunden und gegebenenfalls in entsprechend kleinere Einheiten zerschnitten wird.

11. Verfahren nach Anspruch 10, **dadurch gekennzeichnet**, daß ein Teil der Reagenzien nachträglich in die getrocknete Filmschicht imprägniert wird.

12. Verwendung von filmbeschichteten Geweben oder Vliesen, bei denen eine Filmschicht aus einer Reagenzien und einen Filmbildner enthaltenden Dispersion oder Lösung derartig auf einen Träger aus einem multifilen Gewebe oder Vlies beschichtet ist, daß er sich überwiegend auf einer Seite derselben befindet, zur Herstellung von Teststreifen.


## Claims

1. Test strip with a reagent-containing film layer and a synthetic resin foil serving as handle, characterised in that, for the formation of a filmcoated fabric or fleece, the film layer is coated by application and subsequent drying of a dispersion or solution containing reagents and a film former in such a manner on a carrier of a multifilar fabric or fleece that it is present preponderantly on one side thereof and the film-coated fabric or fleece is fixed on the foil handle.

2. Test strip according to claim 1, characterised in that the fabric or fleece consists of polyester, polyamide, cottonwool or cellulose.

3. Test strip according to one of claims 1 - 2, characterised in that the dry film layer has a thickness of 10 - 200 $\mu$m.

4. Test strip according to claim 3, characterised in that the film layer has a thickness of 15 - 100 $\mu$m.

5. Test strip according to one of claims 1 - 4, characterised in that an additional absorbent layer is present between the handle foil and the film-coated fabric or fleece.

6. Test strip according to claim 5, characterised in that the absorbent layer and the film-coated fabric or fleece are connected by means of a thin mesh which is fixed on the carrier foil adjacent the layer packet.

7. Test strip according to claim 5, characterised in that the handle foil and the absorbent layer are connected with one another.

8. Test strip according to claim 7, characterised in that a covering layer is present above the film-coated fabric or fleece.

9. Test strip according to claim 8, characterised in that the covering layer is transparent and is connected with the film-coated fabric or fleece.

10. Process for the production of test strips according to one of claims 1 - 9, characterised in that a dispersion or solution of the components forming the film layer in a suitable solvent is applied to one side of the fabric or fleece and drivd, whereupon this is connected in per se known manner with the other components of the test strip and is possibly cut up into correspondingly smaller units.

11. Process according to claim 10, characterised in that a part of the reagents is subsequently impregnated into the dried film layer.

12. Use of film-coated fabrics or fleece in which a film layer of a dispersion or solution containing reagents and a film former is coated in such a manner on a carrier of a multifilar fabric or fleece that it is preponderantly present on one side thereof, for the production of test strips.

**Revendications**

1. Bande de test comportant une couche de film contenant un réactif et une feuille en matière plastique servant d'organe de préhension, caractérisée en ce que la couche de film est appliquée sur un support, pour former un tissu ou nappe revêtu d'un film, par application et séchage subséquent d'une dispersion ou solution contenant un réactif et un agent filmant sur un support constitué d'un tissu multifil ou d'une nappe l'application étant réalisée de façon que la couche de film se trouve essentiellement sur une face du support et que le tissu ou nappe revêtu d'un film soit fixé sur la feuille de préhension.

2. Bande de test selon la revendication 1, caractérisée en ce que le tissu ou nappe est constitué de polyester, de polyamide, de coton ou de cellulose.

3. Bande de test selon l'une des revendications 1 ou 2, caractérisée en ce que la couche de film séchée présente une épaisseur de 10-200 μm.

4. Bande de test selon la revendication 3, caractérisée en ce que la couche de film présente une épaisseur de 15-100 μm.

5. Bande de test selon l'une quelconque des revendications 1-4, caractérisée en ce qu'entre la feuille de préhension et le tissu ou nappe revêtu d'un film, l'on dispose une couche supplémentaire absorbante.

6. Bande de test selon la revendication 5, caractérisée en ce que la couche absorbante et le tissu ou nappe revêtu d'un film sont reliés par un filet fin qui est fixé à son tour sur la feuille de support, à côté de l'empilement de couches.

7. Bande de test selon la revendication 5, caractérisée en ce que la feuille de préhension et la couche absorbante sont reliées entre elles.

8. Bande de test selon la revendication 7, caractérisée en ce qu'au-dessus du tissu ou nappe revêtu d'un film, se trouve disposée une couche de couverture.

9. Bande de test selon la revendication 8, caractérisée en ce que la couche de couverture est transparente et reliée au tissu ou nappe revêtu d'un film.

10. Procédé de fabrication d'une bande de test selon l'une quelconque des revendications 1-9, caractérisé en ce qu'une dispersion ou solution des constituants formant la couche de film, dans un solvant approprié, est appliquée sur une face du tissu ou nappe et séché, après quoi elle est liée, de manière connue en soi, aux autres composants de la bande de test, et le cas échéant, découpée en unités plus petites.

11. Procédé selon la revendication 10, caractérisé en ce qu'une partie des réactifs est imprégnée ultérieurement dans la couche de film séchée.

12. Utilisation de tissu ou nappe revêtu d'un film, pour la fabrication de bandes de test, dans laquelle une couche de film, fabricée d'une dispersion ou solution contenant un réactif et un agent filmant, est appliquée sur un support constitué d'un tissu multifil ou d'une nappe, de façon que la couche de film se trouve essentiellement sur une face du support.

Abb. 1

Abb. 2

Abb. 3